# EUROPEAN PATENT APPLICATION

(11) **EP 1 797 890 A1**
(43) Date of publication of application: **20.06.2007**
(21) Application number: 05027005.7
(22) Date of filing: 09.12.2005
(51) Int. Cl.: A61K 35/16, A61K 41/00, A61P 25/08, A61P 25/14, A61P 25/16

(54) **A method for obtaining a biologically active fraction of blood serum treated with gamma irradiation and the use thereof**

(71) Applicant: OWEN Holding LTD, Douglas Isle of Man (GB)
(72) Inventor: Shestakov, Vitaly, 117292 Moscow (RU)
(74) Representative: Zwicker, Jörk

(57) **Abstract**

The present invention relates to a method for preparing a biological active fraction of blood serum, the biological active fraction (S-1-10) and a pharmaceutical composition comprising said fraction as well as uses thereof in the treatment of various diseases and conditions, in particular seizures, cardiovascular diseases and proliferative diseases.

## Description

The present invention relates to a method for preparing a biological active fraction of blood serum, the fraction (S-1-10), and a pharmaceutical composition comprising said fraction as well as uses thereof in the treatment of various diseases and conditions, in particular epileptic seizures, cardiovascular diseases and proliferative diseases.

### Background of the Invention

Methods for the preparation of active substances from blood serum are known, in the art. One is based on the withdrawal of blood from humans or animals, the subsequent incubation as well as the separation of the active substance and finally the preservation of the substance (see, for example, JP 2123287, EP 0 542 303, RU 2096041, RU 2120301). The prior art method concerns the preparation of a blood serum which improves the resistance of the body in respect of exogenic and endogenic factors like air pressure, air temperature, gravity, light etc. as well as hunger, thirst, sleeping and sexual desires etc. The blood serum is drawn from the donor who has previously been brought into a certain functional state and according to the length of the application of the functional state and the type of functional state, e.g. sleep deprivation, alcohol abuse, nicotine abuse etc., blood serum with different biological activity can be obtained which shows mitogenic, somnogenic, opthalmogenic, audio active, thermo active, diatary active, sexually active, anti-hypoxic, anti-alcohol and anti-nicotine activity.

A different method is disclosed in EP 1 283 047 and concerns the treatment of animal blood serum by gamma irradiation with the aim to increase the biological activity of the blood serum product.

Presently, a lot of research focuses on the mechanisms, which regulate cellular proliferation of different human tissues. In this respect both stimulators and inhibitors of proliferation of normal and pathologically abnormal somatic cells, including nerve cells, are investigated (see, for example Aschmarin, I.P. "Neurochemistry", Moskwa, Publishers of the Biomedical Chemical Institute of the Russian Academy of Science", 1996).

It has been observed that peptide growth factors apart from their general activating functions, e.g. stimulation of mitosis, cell differentiation and cell growth of different types of normal tissue, increased wound healing, can cause tumor formation and proliferation (see, for example, Bouneres, P. (1993), Horm. Res. 40: 31; Robinson, C., (1993) Ann. Med. 25: 535; Dignez, C. and Casanueva F. (1995) Trends Endocrin. Metab. 6: 55, Menster D. et al. (1995) Clin. Exp. Metastasis 13: 67).

Such peptides as, for example, the parathyroid peptides, gastrin or bombesin foster the development of tumor cells as well as the development of breast, bone and colon cancer (see, for example, Kitazawa S. and Maeda S. (1995) Clin. Orthop. 312: 45-50 and Kaji et al. (1995) Endocrinology 136: 842).

Although some peptides facilitate normal cell division and are stimulators for human and animals there is the danger that their use will lead to tumor cell development and eventually to the development of cancer.

Earlier experiments have shown that stimulation of animals with electricity leads to an increase of the β-endorphin level in the blood (see, for example, Litvinova S.V. et al. (1990) Biomed. Sci. 5: 471). In a reference work by Udovitschenko, W.I. numerous data is provided with respect to the results of stimulation or shock due to various causes. It has been shown that, for example, electroshock leads to a marked increase of the concentration of β-endorphines, meta- and leu-encephalines within the blood (see Udowitschenko, W.I. (1989) "Xenogenic Opioid System in Shock" Pathiological Physiology and Experimental Therapy" 6: 72-77).

### Summary of the Invention

An object of the present invention was the development of a novel method for the preparation of a biological active fraction of blood serum from animal blood. Surprisingly it was found that the biological active fraction prepared according to the method of the invention exhibited new therapeutic properties.

Consequently, one aspect of the present invention is a method for producing a biological active fraction of blood serum comprising the steps of:
a) withdrawal of blood from an animal,
b) isolation of serum from said blood,
c) gamma irradiation of said serum and
d) selection of a fraction of said serum with a size smaller than 25 kD.

It is further preferred that the animal is electrostimulated prior to step a), in this embodiment the animal is preferably a non-human animal, which is preferably selected form the group consisting of mammals and birds, preferably from poultry, e.g. chicken, duck, turkey, goose, ostrich, and quail. Preferred mammals are dogs, cats, sheep, cattle, horses, donkeys, pigs, goats, and apes.

Although the electrostimulation can be employed to any part of the body it is preferred that the electrostimulation is applied to the head, the neck, the body and/or one or more limbs of the animal. Out of those areas of the body it is preferred that the head of the animal is electrostimulated. In the context of the present invention the terms electrostimulation and electroshock are used interchangeably.

In a preferred embodiment of the method of the present invention the electrostimulation is carried out for a time period of between 1 and 60 seconds, preferably between 1 and 30 seconds, and more preferably between 2 and 10 seconds. It is also preferred that the electrostimulation is carried out with a voltage in the range of between 50 V and 150 V, preferably in the range of between 80 V to 120 V, and more preferably in the range of between 110 V and 120 V. During the performance of the electrostimulation certain currents are preferred and preferably the electrostimulation is carried out with a current in the range of between 0.01 A and 0.4 A, preferably in the range of between 0.02 A and 0.1 A, and more preferably in the range of between 0.04 A and 0.06 A.

In a preferred embodiment of the method of the present invention the electrostimulation is carried out with a frequency in the range of between 10 and 200 Hz, preferably in the range of between 20 to 100 Hz and more preferably in the range of between 45 to 65 Hz.

In a further preferred embodiment of the method of the present invention the gamma irradiation is administered with an adsorbed radiation dose of between 10 to 40 kGy, preferably 15 to 35 kGy and more preferably of between 20 and 30 kGy. The gamma radiation source can be any source, however, a preferred source of gamma radiation is selected from the group consisting of ⁶⁰Co, ¹³⁷Cs, ⁶⁷Cu, ⁶⁷Ga, ¹¹¹In, ¹⁹² Ir, ^{99m}Tc and ¹⁷⁰Tm. Preferably the radiation source is ⁶⁰Co.

When carrying out step d) of the method of the present invention it is preferred that the selection is done using filtration, chromatography, dialysis, centrifugation and/or electrophoresis. It is particularly preferred that the selection is carried out by the use two or more subsequent identical or different selection processes selected from the group consisting of filtration, chromatography, dialysis, centrifugation and/or electrophoresis. The size of the fraction thereby obtained preferably has a size of smaller than 20 kD, more preferably smaller than 15 kD and most preferably smaller than 10 kD.

In a preferred embodiment of the method of the present invention the blood is arterial and/or venous blood.

In a further preferred embodiment of the method of the present invention the method further comprises the step of incubating said blood prior to step b).

In a further preferred embodiment of the method of the present invention the method further comprises the step of lyophilization of said serum prior to step c).

In a further preferred embodiment of the method of the present invention the method further comprises the step of extraction of said serum after step c). It is preferred that the extraction is carried out with a solvent comprising a protease inhibitor.

In a further preferred embodiment of the method of the present invention the method further comprises the step of lyophilization of said serum fraction after step d).

Another aspect of the present invention is the biological active fraction which is producible according to the method of the present invention.

A further aspect of the present invention is a pharmaceutical composition comprising a biological active fraction according to the present invention and one or more pharmaceutically acceptable diluents; carriers; excipients, including fillers, binders, lubricants, glidants, disintegrants, adsorbents; and/or preservatives.

In a preferred embodiment of the pharmaceutical composition of the present invention the composition is formulated as a syrup, an infusion or injection solution, a tablet, a capsule, a capslet, lozenge, a liposome, a suppository, a plaster, a band-aid, a retard capsule, a powder, or a slow release formulation. Preferably the diluent is water, a buffer, a buffered salt solution or a salt solution and the carrier preferably is selected from the group consisting of cocoa butter and vitebesole.

Another aspect of the present invention is the use of the biological active fraction of the present invention or of a pharmaceutical composition of the present invention for the production of a medicament for the improvement of cognitive and/or learning skills in particular improvement of the long term memory.

Another aspect of the present invention is the use of the biological active fraction of the present invention or of a pharmaceutical composition of the present invention for the production of a medicament for the treatment of seizures, in particular epileptic seizures, tremor, in particular tremor associated with Parkinson's disease, proliferative diseases and cardiovascular diseases, in particular myocardial infarction and apoplexy.

In a preferred embodiment of the use of the present invention the proliferative disease is selected from the group consisting of malignomas of the gastrointestinal or colorectal tract, the liver, the pancreas, the kidney, the bladder, the thyroid, the prostate, the endometrium, the cervix, the ovary, the uterus, the testes, the skin, the oral cavity; melanoma; dysplastic oral mucosa; invasive oral cancers; small cell and non-small cell lung carcinomas; mammary tumors, in particular hormone-dependent breast cancers and hormone independent breast cancers; transitional and squamous cell cancers; neurological malignancies including neuroblastomas, gliomas, astrocytomas, osteosarcomas, meningiomas; soft tissue sarcomas; hemangioamas and endocrinological tumors, in particular pituitary adenomas, pheochromocytomas, paragangliomas, haematological malignancies, in particular lymphomas and leukemia.

And in a further preferred embodiment of the use of the present invention the proliferative disease comprises cells similar to the human T cell lymphoma cell line Jurkat, the human B cell lymphoma cell line Raji, the human melanoma cell line Bro, the human cervical cancer cell line HeLa, the human adenocarcinoma cell line MCF-7, the osteosarcoma cell line Mg63, the fibrosarcoma cell line HT1080, the neuroblastoma cell line IMR-32 and the hepatocarcinoma cell line HepG2.

In a further preferred embodiment of the use of the present invention the medicament is administered to a subject in need of treatment in an amount ranging from 0.1 to 50 mg/kg body weight, preferably ranging from 1 to 10 mg/kg body weight.

### Detailed Description of the Invention

The present inventors have surprisingly found that γ-radiation of blood serum and subsequent selection of a fraction of the serum with a size smaller than 25 kD leads to a biological active fraction with improved properties capable of positively influencing various body functions, conditions and diseases of a patient as described in more detail below.

The beneficial effect of a fraction of blood serum produced by treating blood serum with γ-radiation and size selection and optionally, with electroshock, is surprising for two reasons. Firstly, it was discovered that blood and serum prepared from blood is sensitive to radiation and is unstable and deactivated under ionizing radiation (see, for example, Radiomedicine - M. Atomisdat (1972) 123-125 and Gergely, J. et al. (1967) Radiosterilization of Medical Products 115-124). Secondly, it is known in the prior art that electroshocks cause severe disturbances of all vital functions and systems within the organism in particular the central nervous system, the blood and circulatory system and the respiratory system (see, for example, Orlow, A.N. et al. (1977) Medicine*).*

These studies did not lead to the results of the present invention and would have suggested that it would not be possible to obtain a biological active fraction of blood serum through the combination of γ-radiation treatment and fractionation and optionally electroshock. Thus, it was surprising that arterial and/or venous blood drawn from an animal, in particular from a chicken treated with gamma radiation and which was further size selected for components with a size of smaller than 25 kD resulted in a biological active fraction. The specific activities of the thus obtained biological active fraction will be set out in more detail below.

Accordingly, a first aspect of the present invention is a method for producing a biological active fraction of blood serum comprising the steps of:
a) withdrawal of blood from an animal,
b) isolation of serum from said blood,
c) gamma irradiation of said serum, and
d) selection of a fraction of said serum with a size smaller than 25 kD.

It is further preferred that the animal is electrostimulated prior to step a), in this embodiment the animal is preferably a non-human animal. Various animals can be used in the method of the present invention, however, it is preferred that the non-human animal is selected from the group consisting of mammals and birds. Because of their easy availability it is particularly preferred to use farm animals like poultry, e.g. chicken, duck, turkey, goose, ostrich and quail. A particular preferred animal which can be used in the method of the present invention is a chicken. The type of mammal that can be used in the method of the present invention is not particularly restricted and comprises without limitation rodents and farm animals, e.g. mice, hamsters, rats, cats, dogs, horses, donkeys, sheep, cows, pigs and goats.

It is envisioned that the electrostimulation leads to the release of certain compounds within the animal which cause and/or contribute to the surprising therapeutic effect of the biological active fraction of the present invention. The non-human animal can be stimulated in different regions of the body. Preferably the electrostimulation is carried out at the head, the neck, the body and/or on one or more of the limbs. It is possible to stimulate the body only at one position or at several positions at once. A particular preferred body part for the electrostimulation is the head of the respective animal. When stimulating birds, in particular chicken it is preferred that the head is electrostimulated.

The electrostimulation can be carried out by any art known method including but not limited to using metal electrodes or water baths as used, for example, during culling of cattle or electrocution of poultry. Preferably, the electrostimulation is carried out for a time period of between 1 and 60 seconds, preferably between 1 and 30 seconds, more preferably between 2 and 10 seconds, and most preferably between 3 and 4 seconds. The length of a time period will be longer in case that a large animal is electrostimulated and can be shorter in cases were small animals are electrostimulated. For example for the stimulation of chicken, in particular heads a preferred time period of the electrostimulation is between 2 and 10 seconds and more preferably between 3 and 4 seconds.

The other variables which can be adapted during the electrostimulation of the animal is the voltage, the current and the frequency of the current and the present inventors have defined certain preferred ranges for these parameters as set out below. The actual parameter chosen will depend in part on the size of the animal as well as on the region of the animal to be stimulated. In general larger animals and larger regions will require a higher voltage and current. Thus, the electrostimulation is preferably carried out with a voltage in the range of between 50 Volt and 150 Volt, preferably 80 Volt to 120 Volt and more preferably between 110 Volt and 120 Volt. The ranges for the currents that can be applied are between 0.01 A and 0.4 A, preferably between 0.02 A and 0.1 A, more preferably between 0.04 A and 0.06 A and most preferably about 0.05 A. Voltage, current and application time are preferably chosen to administer energy in the range of between 1 Ws and 1,000 Ws, preferably in the range of 10 Ws to 200 Ws and even more preferably in the range of 15 Ws to 100 Ws.

For the stimulation of the preferred animals, i.e. chicken, it is preferred that the electrostimulation is carried out with a voltage in the range of between 80 Volt to 120 Volt and more preferably between 110 Volt and 120 Volt. Furthermore, a current in the range of between 0.04 A and 0.06 A, in particular of 0.05 A is preferred in the context of the electrostimulation of birds, in particular of chicken.

In a particular preferred embodiment of the method of the present invention the electrostimulation of a bird, in particular a chicken, is carried out preferably at the head for between 2 and 10 seconds, preferably for between 3 and 4 seconds at a voltage of between 80 V and 120 V, in particular of between 110 V and 120 V. In this preferred embodiment the current is preferably between 0.04 A and 0.06 A and most preferably about 0.05 A, i.e. energy in the range of 10 Ws to 30 Ws, preferably 16 Ws to 24 Ws is administered to the bird.

The frequency of the electrostimulation does not appear to be particularly critical but is pref erably in the range of between 10 and 200 Hertz, more preferably in the range of between 45 to 65 Hz and most preferably around 50 Hz.

The gamma irradiation of the serum during step c) of the method of the present invention can be carried out with any gamma source including X-ray sources and radionuclides. Preferably the gamma irradiation source is a radionuclide with a defined gamma irradiation pattern. Preferred sources for the gamma irradiation are selected from the group consisting of ⁶⁰Co, ¹³⁷Cs, ⁶⁷Cu,⁶⁷Ca, ¹¹¹In, ¹⁹²Ir, ^{99m}Tc and ¹⁷⁰Tm. Out of those ⁶⁰Co, ¹³⁷C_{S}, ¹⁹²Ir and ¹⁷⁰Tm are particular preferred with ⁶⁰Co being the most preferred gamma radiation source for use in the method of the present invention. The radiation dose adsorbed by the serum is preferably in the range of between 10 to 40 kGy preferably in the range of between 15 to 35 kGy and more preferably in the range of between 20 and 30 kGy, i.e. 25 ± 5 kGy. Gamma irradiation on one hand sterilizes the serum and the other bolsters the activity of the serum fraction. The suitable irradiation time can vary in a wide range, depending on the desired level of activity of the resulting serum fraction and the respective indication for which the serum fraction is to be used. The experimental section describes assays to test the activity of the serum fraction of the invention and these tests can be used by someone of skill in the art to determine the optimal duration of the application of irradiation without an undue burden. Preferably the irradiation is carried out for a period of time in the range of 30 min to 10 hours, preferably for 45 min to 8 h and more preferably for 1 h to 5 h, i.e. for 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295 or 300 min. It is further preferred that the energy dose applied for this period of time is within a range of 10 to 40 kGy, preferably 15 to 35 kGy and more preferably between about 20 to 30 kGy. Again the preferred radiation source in this context is ⁶⁰CO.

Various methods are known in the art to select proteinaceous and non-proteinaceous components based on their molecular weight and molecular size, respectively. The size of the selected fraction can be determined with any of a number of art known methods including without limitation denaturing or native SDS-PAGE including peptide standards of known size and mass spectrometry (MS). A fraction is considered to have a size of 25, 20, 15, and 10 kD, respectively, or less if at least 70%, preferably at least 80%, more preferably at least 90%, more preferably at least 95%, more preferably at least 99% and most preferably 100% of the components, preferably the protein components in the fraction, have a size below the respective size threshold.

Preferably, the selection is carried out using filtration, chromatography, dialysis, centrifugation and/or electrophoresis. Filters of various pore sizes are commercially available. Typically a filter usable in the method of the present invention consists of a housing enclosing a membrane, which is made of, e.g. nylon, teflon^{®}, polyurethane, polyvinylchloride, polypropylene, polysaccharides or the like or a combination thereof. The pore size is chosen to prevent the passing of molecules with a molecular mass larger than about 25 kD, preferably larger than 20, more preferably larger than 15 kD and most preferably larger than 10 kD. Filters with various molecular weight cut offs are commercially available from, e.g. Millipore, which of fers ultrafiltration (UF) membranes in a variety of filter formats including Minitan plates, Pellicon cassettes, Helican-UF-cartrdiges and Prostak UF modules. The respective choice of filtration device will depend on the amount of serum to be filtered and can be appropriately selected by someone of skill in the art. The solution to be filtered can be applied to the filter by gravity, by positive or by negative force, including centrifugation of the serum in so called spin columns. Preferably, the serum is filtered with the application of a positive force. Prior to filtration it is preferred that the serum to be filtered is spun in a centrifuge, to remove small particles or particulates which otherwise would clog the filter. Preferably the serum is spun until all turbidity is removed. This occurs when spinning the serum or a suspension of the lyophilized serum (in case that the gamma irradiation was carried out on lyophilized serum), e.g. for 10 min to 24 h at a speed of 5,000 to 25,000 rpm, preferably for 10 min to 2 h at a speed of 8,000 to 15,000 rpm. A preferred filter is a filter with a pore size smaller than 5 µm, preferably smaller than 2 µm, and even more preferably with a pore size 0.45 µm or smaller, which are commercially available from a number of sources including Millipore.

Chromatographic methods for the selection of proteins of a particular size and molecular mass, respectively, include size exclusion and gel filtration columns. These columns trap molecules smaller than the pore openings of the chromatographic material in the chromatographic material, while larger molecules flow through the column. A large variety of size exclusion columns with different separation ranges are commercially available. Someone of skill in the art can easily chose an appropriate column to obtain a fraction of the blood serum with the above indicated molecular mass and preferred molecular mass.

Another approach for selecting components of a certain molecular mass and size involves dialysis. Dialysis membranes are available with a variety of pore sizes. Upon dialysis of a blood serum enclosed in a dialysis membrane, which only allows molecules of a size smaller than 25 kD to pass through the membrane into the dialysis solution, the dialysis solution will after dialysis comprise the majority of the molecules with a size smaller than 25 kD. The dialysis solution can then be concentrated by art known methods including lyophilization, ultrafiltration and the like.

It is also possible to carry out the separation of proteins using centrifugation. To that end the protein solution can be applied to a gradient of, for example, CsCl, sucrose or the like. The gradient can either be preformed or can be established during centrifugation. Preferably centrifugation is carried out in a swinging bucket or fixed angle type of rotor. The various serum components will upon centrifugation migrate to a region within the centrifuge tube, which reflects their size and molecular mass, respectively. The smaller molecules will be at the top of the centrifuge tube, i.e. closer to the axis of the rotor, while the larger will be at the bottom. Accordingly, the withdrawal of the toplayers of solution from the centrifuge tube after centrifugation will yield the small components of the blood serum. To determine the fraction comprising components smaller than, e.g. 25 kD it is possible to include, e.g. a fluorescently or radioactively labelled protein weight standard with a size of 25 kD, 20 kD, 15 or 10 kD, which will migrate to the lower end of the fraction to be withdrawn and can be detected. Consequently, withdrawal of the solution above the respectively used weight standard will lead to a fraction with the desired size range. It is preferred that the centrifugation is carried out under reducing conditions to break up sulfid links, which can be between and/or within protein chain(s) in order to allow the proteins to migrate to regions within the gradient, which more accurately reflects their respective mass.

Electrophoresis is a further way of separating proteins. Electrophoresis is preferably carried out on a polymeric matrix like, e.g. polyacrylamide or agar. Preferably, electrophoresis under denaturing and reducing conditions is employed, which allows a better separation of proteinaceous compounds according to their molecular weight rather than according to their size. A preferred electrophoretic separation method is denaturing SDS-polyacrylamide gel electrophoresis (SDS-PAGE). This and other methods of carrying out electrophoresis of proteins are well known in the art and are described in standard textbooks like, for example, Sambrook J., et al. Molecular Cloning: A Laboratory Manual, 3. Volumes, 3rd Edition, 2001, Cold Spring Harbor Laboratory.

In a preferred embodiment the separation is carried out by two or more subsequent identical or different selection processes selected from the group consisting of filtration, chromatography, dialysis, centrifugation and/or electrophoresis. For example, a first selection step can be a preselection, which selects all molecules smaller than, e.g. 1000 kD. This allows removing, e.g. high molecular weight components, undissolved particles etc., which might otherwise interfere with the selection step used for the selection of the fraction of the blood serum with a size smaller than 25 kD. In particular when using a filter as the first selection step a filter with a large pore size is used, e.g. with a diameter of, e.g. 5 µm, 2 µm, or 0.45 µm to separate high molecular weight compounds and larger undissolved particles from the serum. A filter with a size of 0.45 µm is preferred in a first separation step. Subsequently, a filter with a pore-size which selects molecules with a molecular weight of 25 kD, preferably 20 kD, more preferably 15 kD and most preferably 10 kD or less is employed.

The fraction of the blood serum selected with one or more of above selection processes, preferably with two filtration steps has a size of smaller than 25 kD, preferably 20 kD, preferably smaller than 15 kD and more preferably smaller than 10 kD.

The withdrawal of the blood from the animal can be effected by any art known method and includes syringes as well as puncturing of arteries or veins or decapitation in particular in the context of the withdrawal of blood from birds. It is possible to withdraw only a part of the blood or to completely withdraw the blood of the animal. The later is preferably used, if a lethal dose of electricity has been applied to the animal. The withdrawn blood can be arterial and/or venous blood.

The serum can be isolated from the blood by any art known method including filtration, sedimentation and centrifugation. It is, however, preferred that the blood is incubated for between 4 and 72 h at a low temperature, e.g. between 2° and 10°C, preferably between 4 and 8°C to allow clotting of the blood which leads to the release of additional factors into the serum. Thus, in a preferred embodiment of the method of the present invention the method further comprises the step of incubating the blood after the withdrawal of the blood from the animal and prior to the isolation of the serum from the blood, e.g. for between 4 and 72 h at a low temperature, e.g. between 2° and 10°C, preferably between 4 and 8°C.

In a further preferred embodiment of the method of the present invention the method comprises the further step of lyophilization of the serum prior to the irradiation step c). The lyophilization allows easier handling of the serum during irradiation and optimizes absorption of the radiation by the serum components. Alternatively, it is also possible to lyophilize the serum after irradiation, if required. The lyophilized blood serum has to be dissolved again prior to carrying out the filtration step d).

The lyophilized blood serum comprises some components, which are poorly soluble in aqueous solvents, however, it was found that the components of the blood serum, which are soluble in an aqueous solution comprise the majority of the active principle of the blood serum. Thus, in a preferred embodiment of the method of the present invention the method comprises the further step of one or more extractions of said serum after step c) and preferably prior to step d). The extraction is effected preferably by suspending the lyophilized blood serum in an aqueous solvent, in particular water, a biologically compatible buffer, e.g. Hepes, Tris-HCl, citrate or phosphate buffer, a salt solution, e.g. NaCl, or a buffered salt solution, e.g. Ringer's solution or phosphate buffered saline (PBS) and the subsequent separation of the aqueous fraction from the undissolved components preferably through centrifugation. Preferably the extraction is carried out with water. It is also preferred that the extraction process is carried out at least twice. The resultant aqueous solubilized blood serum fraction can be concentrated by any art known method including lyophilization and filtration.

To prevent degradation of the proteinaceous compounds during the extraction as well as during subsequent manipulations it is preferred that a protease inhibitor is added to the solvent. Preferred protease inhibitors are 4-(2-aminoethyl)benzenesulfonyl fluoride (AEBSF), antipain, aprotinin, benzamidine, bestatin, chymostatin, E-64, ε-aminocaproic acid (EAC), ethylenediaminetetraacetic acid disodium salt (EDTA), ethylene glycol-bis(2-aminoethylether)-N,N,N',N'-tetraacetic acid, leupeptin hemisulfate, leupeptin hydrochloride, N-ethylmaleimide (NEM), pepstatin A, phenylmethanesulfonylfluoride (PMSF), phosphoramidon disodium salt, 1,10-phenanthroline monohydrate, and trypsin inhibitor. In many cases it will be preferred to use a so called protease inhibitor cocktail, which comprises several different protease inhibitors with different specificities, e.g. specificities for serine, cysteine, aspartic proteases and aminopeptidases. Suitable protease inhibitor cocktails are available from various commercial sources, including, e.g. INHIB-1 from Sigma, which comprises AEBSF, antipain, aprotinin, benzamidine, bestatin, chymostatin, E-64, EAC, EDTA, leupeptin, NEM, pepstatin A, phosphoramidon, and trypsin inhibitor. A preferred protease inhibitor is PMSF.

Once the size selection, preferably by a two step filtration of the blood serum has been carried out it is preferred that the filtrate is lyophilized for easier storage and handling and, thus, the method in a preferred embodiment further comprises the step of lyophilization of the selected serum fraction after step d).

A preferred mode of carrying out the method of the present invention comprises the following steps:
a) electrostimulation of the head of a bird, preferably a chicken with between 80 V to 120 V, preferably 110 V to 120 V, at a current of between 0.04 to 0.06 A, preferably 0.05 A, for between 2 and 10 seconds, preferably for 3 to 4 seconds,
b) withdrawal of blood from said bird,
c) incubation of said blood until clot formation, e.g. between 4 and 72 h at between 2°C to 10°C,
d) isolation of serum from said blood,
e) optionally lyophilization,
f) gamma irradiation of said serum with between 20 to 30 kGy for 3 to 4 h whereby the gamma radiation source is preferably ⁶⁰Co,
g) optionally lyophilization and for one or more times extraction of the components of the serum, which are soluble in an aqueous solvent, which is preferably water, and
h) selection of a fraction of said serum and aqueous serum extract, respectively, with a size smaller than 25 kD, preferably 20 kD, more preferably 15 kD and most preferably 10 kD or less preferably using at least two subsequent filtration steps, wherein the first filter has a larger pore size than the second filter.

A further aspect of the present invention is the biological active fraction itself, which is producible according to the method of the present invention. It is distinct from prior art blood serums which do not employ the steps of the method of the present invention and, thus, do not provide a biological active fraction of blood serum.

It has been surprisingly found that the biological active fraction of the present invention provides certain therapeutic effects, e.g. an anti-proliferative activity or an anti epileptic activity, therefore a further aspect of the present invention is a pharmaceutical composition, comprising a biological active fraction producible according to the method of the present invention. Such a pharmaceutical composition can further comprise one or more pharmaceutically acceptable diluents; carriers; excipients, including fillers, binders, lubricants, glidants, disintegrants, and adsorbents; and/or preservatives.

The pharmaceutical composition of the present invention can be administered by various well known routs, including oral, rectal and parenteral administration, e.g. intravenous, intramuscular, intranasal, intradermal, subcutaneous and similar administration routes. Parenteral administration and particular intravenous administration is preferred. Depending on the route of administration different pharmaceutical formulations are required and some of those may require that protective coatings are applied to the drug formulation to prevent degradation of the biological active serum in, for example, the digestive tract.

Thus, preferably the pharmaceutical composition of the present invention is formulated as a syrup, an infusion solution, or injection solution, a tablet, a capsule, a capslet, a lozenge, liposome, a suppository, a plaster, a band-aid, a retard capsule, a powder or a slow release formulation.

Particular preferred pharmaceutical forms are forms suitable for injectionable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water-buffered aqueous solutions, e.g. biocompatible buffers, ethanol, polyol, such as glycerol, propylene glycol, polyethylene glycol, suitable mixtures thereof, surfactants or vegetable oils. The biological active fraction of the present invention can also be formulated into liposomes, in particular for parenteral administration. Liposomes provide the advantage of increased half life in the circulation, if compared to the free drug and a prolonged more even release of the enclosed drug.

Sterilization of infusion or injection solutions can be accomplished by any number of art recognized techniques including but not limited to addition of preservatives like anti-bacterial or anti-fungal agents, e.g. parabene, chlorobutanol, phenol, sorbic acid or thimersal. Further, isotonic agents, such as sugars or salts, in particular sodium chloride may be incorporated in infusion or injection solutions.

Production of sterile injectable solutions containing the biological active fraction is accomplished by incorporating the biological active serum in the required amount in the appropriate solvent with various ingredients enumerated above as required followed by sterilization. To obtain a sterile powder the above solutions are lyophilized by art known methods, including vacuum-drying or freeze-drying as necessary. Preferred diluents of the present invention are water, physiological acceptable buffers, physiological acceptable buffer-salt solutions or salt solutions.

Preferred carriers of the present invention are cocoa butter and vitebesole. Excipients which can be used with the various pharmaceutical forms of the biological active fraction can be chosen from the following non-limiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like;
b) lubricants such as magnesium stearate, talc, calcium stearate, zinc stearate, stearic acid, hydrogenated vegetable oil, leucine, glycerids and sodium stearyl fumarates,
c) disintegrants such as starches, croscaramellose, sodium methyl cellulose, agar, bentonite, alginic acid, carboxymethyl cellulose, polyvinyl pyrrolidone and the like.

Other suitable excipients can be found in the Handbook of Pharmaceutical Excipients, published by the American Pharmaceutical Association, which is herein incorporated by reference.

Another aspect of the present invention is the use of the biological active fraction or of a pharmaceutical composition of the present invention for the production of a medicament for the improvement of cognitive and/or learning skills of the treated subject and in particular the improvement of the long-term memory. The usefulness for this indication is based on the discovery that the biological active fraction or the pharmaceutical composition of the present invention increases the learning abilities of treated subjects.

Furthermore, the blood serum or the pharmaceutical composition can be used for the treatment of seizures of any type. In particular, however, for the treatment of epileptic seizures. In particular in severe epileptic forms and during grand mal seizures the administration of the biological active fraction or of the pharmaceutical composition of the present invention can prevent death that is sometimes associated with severe seizures.

Another possible use of the blood serum or the pharmaceutical composition is the use for the treatment of tremor. Tremor of various severities is associated with a number of diseases and conditions including side effects of chemicals or drugs. Preferably the tremor to be treated is tremor mercurialis, orthostatic tremor, psychogenic tremor, lead tremor, senile tremor, toxic tremor, cerebellar tremor, essential tremor and tremor associated with Parkinson's diseases, most preferably the tremor associated with Parkinson's disease.

A further aspect of the present invention is the use of the biological active fraction or of the pharmaceutical composition of the present invention for the production of a medicament for the treatment of proliferative diseases and cardiovascular diseases, in particular myocardial infarction and apoplexy.

It was particular surprising that the biological active fraction or the pharmaceutical composition of the present invention did show a marked anti-proliferative effect, if tested on a variety of tumor cell lines *in vitro.* It, therefore, appears that the biological active fraction or the pharmaceutical composition of the present invention is particular suitable for the treatment of proliferative diseases and preferred proliferative diseases which are treatable according to the use of the present invention are selected from the group consisting of malignomas of the gastrointestinal or colorectal tract, the liver, the pancreas, the kidney, the bladder, the thyroid, the prostate, the endometrium, the cervix, the ovary, the uterus, the testes, the skin, the oral cavity; melanoma; dysplastic oral mucosa; invasive oral cancers; small cell and non-small cell lung carcinomas; mammary tumors, in particular hormone-dependent breast cancers and hormone independent breast cancers; transitional and squamous cell cancers; neurological malignancies including neuroblastomas, gliomas, astrocytomas, osteosarcomas, meningiomas; soft tissue sarcomas; hemangioamas and endocrinological tumors, in particular pituitary adenomas, pheochromocytomas, paragangliomas, haematological malignancies , in particular lymphomas and leukemia.

Since the anti-proliferative effect of the biological active fraction of the present invention or of the pharmaceutical composition of the present invention has been first established for a variety of tumor cell lines it is particular suitable for the treatment of proliferative diseases which comprise cells and/or tumor tissue comprising cells similar to the tumor cell lines used in those experiments. Accordingly, preferred proliferative diseases treatable with the biological active fraction or the pharmaceutical composition comprising the biological active serum comprise cells similar to the human T cell lymphoma cell line Jurkat, the human B cell lymphoma cell line Raji, the human melanoma cell line Bro, the human cervical cancer cell line HeLa, the human adenocarcinoma cell line MCF-7, the osteosarcoma cell line Mg63, the fibrosarcoma cell line HT1080, the neuroblastoma cell line IMR-32 and the hepatocarcinoma cell line HepG2. In this context the term "similar cells" refers to cells, which have the same origin, e.g. T-cell, B cell or neural lineage, as the respective cell line and which carry a mutation in the same or a functionally equivalent gene and wherein this mutation contributes to the proliferative activity of the cell, e.g. mutation in p53, pRb, cdc 2, cdk 4, cyclin A, cyclin B, p21^{ras}, c-fos, c-jun, p107, p130, EGFR, p16^{INK}, and the like; which carry the same or a functionally similar exogenous gene, e.g. human papilloma virus (HPV), E 6 or E 7, insertion into the cyclin B promoter by hepatitis B virus and the like; or which have the same chromosomal rearrangement or abnormality, e.g. deletion, chromosomal multiplicity etc.

Based on the results in animals and in cell culture certain amounts of the biological active fraction are preferred for the treatment of the diseases and conditions for which the fraction and pharmaceutical composition can be employed. It is, however, understood that depending on the respective condition as well as on the respective patient to be treated, i.e. depending on the severity of the disease or condition, the general health status of the patient, etc., different doses of the biological active fraction or the pharmaceutical composition comprising the active fraction are required to elicit a therapeutic effect. The determination of the appropriate dose lies within the discretion of the attending physician. It is contemplated that the dosage of the biological active fraction in the therapeutic method of the invention should be in the range of about 0.1 mg to about 50 mg biological active fraction per kg body weight. However, in a preferred use of the present invention the biological active fraction is administered to a subject in need thereof in an amount ranging from 1 to 25 mg/kg body weight, preferably ranging from 1 to 10 mg/kg body weight. The duration of therapy with the biological active fraction will vary, depending on the severity of the disease being treated and the condition and idiosyncratic response of each individual patient.

The following examples are included to demonstrate preferred embodiments of the invention. It should be appreciated by those of skill in the art that the techniques disclosed in the examples that follow represent techniques discovered by the inventors to function well in the practice of the invention, and thus, can be considered preferred modes for its practise. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Example 1

### Method for obtaining chicken blood treated by electroshock

For the preparation of serum from chicken, the chicken were treated with an electroshock of grade II to III (electrical voltage 70-80 V, current 0.05 A, frequency 50 Hz, application time: 2 to 3 sec at the head). Blood was then drawn from the arteria carotis and further incubated at a temperature of 4 to 8°C for 18 to 24 h in polyethylene flasks. After complete retraction of blood clots the flasks were spun at 3.000 rpm for between 20 to 30 minutes. The serum was separated from the blood clots and lyophilized under art known conditions. The flasks with the lyophilized serum were treated on a RZ-100-M apparatus with 20 to 30 kGy, preferably at around 25 ± 5kGy using ⁶⁰Co as a gamma radiation source for 3 to 4 h. The treated serum was stored at a temperature of between 4 to 8°C for later use.

The lyophilized blood serum was extracted. To this end water comprising 0.5 mmol/l PMSF was added to the serum in a relation of 1:10 (v/v) and incubated under agitation for 1 h at 5°C. The resulting suspension was spun at 12,000 rpm for 30 min in a standard lab centrifuge. The remaining solids were extracted for a second time with water comprising 0,5 mmol/l PMSF for 30 min at 4°C. The suspension was spun again at 12,000 rpm for 30 min and the two supematants were combined. The supernatant was filtered though a filter with a 0.45 µm pore size (Millipore). The flowthrough was subjected to a second filtration step using a filter membrane with a cut-off value of 10 kD (0.45 µm pore size Millipore). The final filtrate was lyophilized using standard methods and was stored until further use at between 4 and 8°C.

### Evidence for the stimulating effect of a fraction of chicken blood serum treated with electroshock

The below experiments were carried out using 40 Wistar rats with an average body mass of 180-200 g. The animals were randomly assigned to 4 groups of 10 rats each. The first group was administered with 1.0 ml of a physiological salt solution. The second, third and fourth group was administered a dose of 0.1; 1.0; 50 mg/kg body weight of the fraction of the present invention within 1.0 ml solution. 30 minutes after the injection the animals, which had a weight attached to their tail (10% of the body weight of the rat), were placed in a basin with water (25°C). The time until the first signs of agony of the animals appeared was measured. The results are shown in Table 1 below.

**Table 1**

| **Influence of the biological active fraction on the perseverance of the rats in min.** | | | | |
|---|---|---|---|---|
| Statistical data | Control | Dose of the fraction (mg/kg body weight) | | |
| | | 0.1 | 1.0 | 50 |
| M+m | 3'31 ± 24" | 4'23 ± 26" | 4'36 ± 29" | 5'27±36" |
| p | | > 0.05 | >0.05 | <0.05 |

| | | | | |
|---|---|---|---|---|
| A dose of 50 mg/kg increased the physical activity of the treated animals in comparison to the control group by 54%. | | | | |

### Example 2

### Parkinson's disease

When modelling Parkinson's disease the 6-GD analogue oxitremorin was used, which induces conditions typical in Parkinson's disease in animals, i.e. a tremor of the extremities, a latency period until onset of the general tremor (of the whole body), periods of lack of tremor as well as the end of the tremor.

Oxitremorin was injected at a dose of 1.0 mg/kg body weight in a physiological salt solution in an amount of 1.0 ml. The biological active fraction was administered at a dose of 0.1, 1.0, 10.0 and 50 mg/kg body weight also diluted with 1 ml of a physiological salt solution. The fraction was applied 45 minutes prior to the administration of oxitremorin. The experiment was carried out using 49 Wistar rats with an average body mass of 100-220 g. The results are depicted in Table 2.

**Table 2**

| **Influence of the biological active fraction on the tremor associated with Parkenson's disease (mean ± error of the means)** | | | | | |
|---|---|---|---|---|---|
| **Characteristic of the tremor** | **Control** | **Dose of the fraction (mg/kg Body weight)** | | | |
| | | **0.1** | **1.0** | **10.0** | **50.0** |
| 1. Latency of the onset of the tremor of the extremities (sec.) | 64.4 ± 3.8 | 85.0 ± 7.1 > 0.05 | 92.8 ± 7.3 < 0.05 | 126.4 ± 17.7 < 0.01 | 157.1 ± 26 |
| 2. Latency of the onset of the general tremor of the animal (sec.) | 216.4 ± 21.7 | 294.4 ± 19.9 | Absent | Absent | Absent |
| 3. Lack of the periods of general tremor (min.) | 7.4 ± 21.7 | 6.7 ± 1.0 | Absent | Absent | Absent |
| 4. End of the tremor of the extremities (min.) | 32.1 ± 5.7 | 21.3 ± 2.5 < 0.05 | 21.2 ± 2.3 < 0.05 | 23.1 ± 3.1 < 0.05 | 14.9 ± 4.0 < 0.01 |

The administration of the biological active fraction at a dose of 0.1 mg/kg body weight, 45 minutes prior to the induction of the symptoms of Parkinson's disease, delayed the onset of the general tremor (tremor of the extremities and the body) by 31 % and decreased the time until the tremor of the extremities ended in comparison to the control group by 50%. A dose in a range of 1.0 mg/kg up to 50 mg/kg body weight showed the strongest effect because it not only decreased the latency time until onset of the tremor of the extremities two fold (starting at a dose of 10 mg/kg) but also decreased the time until the end of the tremor of the extremities was observed to half in comparison to the control group. Notably no general tremor was observed at all, when using a dose of the biological active fraction of 1.0; 10.0 or 50.0 mg/kg body weight.

### Example 3

### Epilepsy

It is known in the state of the art that camphor at a toxic dosage leads to hyperactivation of the motoric area of the central nervous system which in turn causes the development of tonic cramps. Because of this camphor is used besides carazole in animal models for the generation of cramps. Dependent on the doses of the administered camphor it is possible to cause all aspects of a small and large epileptic seizure including grand mal seizures.

In the experiments the influence of the biological active fraction on the epileptic activity, which was induced by injecting camphor into the peritoneal cavity of rats was investigated. 50 male Wistar rats with a weight of 220-230 g were used in this experiment. The animals were randomly assigned to 5 groups of 10 rats each. The biological active fraction was administered to the second through fifth group at a dose of 0.1, 1.0, 10.0 and 50.0 mg/kg body weight, respectively, in an amount of 1 ml of a physiological salt solution. 45 minutes thereafter a solution of camphor oil (20%) was injected into the peritoneal cavity in an amount 0.5 ml.

The seizures were observed and assessed by experts. The following parameter was assessed: The latency time of the reaction, i.e. to time until onset of tonic and clonic cramps, respectively. The results are summarized in Table 3.

**Table 3**

| **Influence of the biological active fraction on experimentally in used epileptic seizures** | | | | | |
|---|---|---|---|---|---|
| **Type of seizures** | **Control (camphor)** | **Dose of the fraction (mg/kg body weight)** | | | |
| | | **0.1** | **1.0** | **10.0** | **50.0** |
| 1. Onset of tonic cramps (min.) | 6.7 ± 0.8 | 7.1 ± 1.1 > 0.05 | 9.0 ± 1.0 < 0.05 | 10.4 ± 1.1 < 0.01 | 11.0 ± 1 |
| Onset of clonic cramps (min.) | 14.3 ± 1.0 | 15.7 ± 1.6 > 0.05 | 18.7 ± 1.4 < 0.05 | 20.0 ± 1.9 < 0.01 | 20.6 ± 1 < 0.01 |

The injection of the biological active fraction at a dose from 1.0 to 50.0 mg/kg body weight of the animal 45 Minutes prior to initiation of the symptoms of epilepsy by injection of camphor extended the latency time until onset of tonic and clonic cramps by 30 to 50%, i.e. the severity of the cramps during epilepsy were significantly reduced.

## Claims

1. Method for producing a biological active fraction of blood serum comprising the steps of:
a) withdrawal of blood from an animal,
b) isolation of serum from said blood,
c) gamma irradiation of said serum, and
d) selection of a fraction of said serum with a size smaller than 25 kD.

2. Method according to claim 1, wherein the animal is treated with electrostimulation prior to step a).

3. Method according to claim 1 or 2, wherein the animal is selected from the group consisting of mammals and birds.

4. Method according to claim 3, wherein the bird is selected from the group consisting of chicken, duck, turkey, goose, ostrich, and quail.

5. Method according to any one of claims 2 to 4, wherein in the electrostimulation is carried out at the head, the neck, the body and/or one or more limbs, preferably at the head.

6. Method according to any one of claims 2 to 5, wherein the electrostimulation is carried out for a time period of between 1 and 60 seconds, preferably between 1 and 30 seconds, and more preferably between 2 and 10 seconds.

7. Method according to any one of claims 2 to 6, wherein the electrostimulation is carried out with a voltage in the range of between 50 V and 150 V, preferably 80 V to 120 V, and more preferably between 110 V and 120 V.

8. Method according to any one of claims 2 to 7, wherein the electrostimulation is carried out with a current in the range of between 0.01 A and 0.4 A, preferably between 0.02 A and 0.1 A, and more preferably between 0.04 A and 0.06 A.

9. Method according to any one of claims 2 to 8, wherein the electro stimulation is carried out with a frequency in the range of between 10 and 200 Hz, preferably in the range of between 20 to 100 Hz and more preferably in the range of between 45 to 65 Hz.

10. Method according to any one of claims 1 to 9, wherein said gamma irradiation is administered with an adsorbed radiation dose of between 15 to 35 kGy, preferably of between 20 and 30 kGy.

11. Method according to any one of claims 1 to 10, wherein the source of the gamma radiation is selected from the group consisting of ⁶⁰Co, ¹³⁷Cs, ¹⁷Cu, ⁶⁷Ga, ¹¹¹In, ¹⁹²Ir, ^{99m}Tc and ¹⁷⁰Tm.

12. Method according to any one of claims 1 to 11, wherein the selection is carried out using filtration, chromatography, dialysis, centrifugation and/or electrophoresis.

13. Method according to any one of claims 1 to 12, wherein the selection is carried out by two or more subsequent identical or different selection processes selected from the group consisting of filtration, chromatography, dialysis, centrifugation and/or electrophoresis.

14. Method according to any one of claims 1 to 13, wherein the fraction has a size of smaller than 20 kD, preferably smaller than 15 kD and more preferably smaller than 10 kD.

15. Method according to any one of claims 1 to 14, wherein said blood is arterial and/or venous blood.

16. Method according to any one of claims 1 to 15, wherein the method further comprises the step of incubating said blood prior to step b)

17. Method according to any one of claims 1 to 16, wherein the method further comprises the step of lyophilization of said serum prior to step c)

18. Method according to any one of claims 1 to 17, wherein the method further comprises the step of extraction of said serum after step c).

19. Method according to claim 18, wherein the extraction is carried out with a solvent comprising a protease inhibitor.

20. Method according to any one of claims 1 to 19, wherein the method further comprises the step of lyophilization of said serum fraction after step d).

21. Biological active fraction producible according to a method according to any one of claims 1 to 20.

22. Pharmaceutical composition comprising a biological active fraction according to claim 21 and one or more pharmaceutically acceptable diluents; carriers; excipients, including fillers, binders, lubricants, glidants, disintegrants, adsorbents; and/or preservatives.

23. Pharmaceutical composition according to claim 22, wherein the composition is formulated as a syrup, an infusion or injection solution, a tablet, a capsule, a capslet, lozenge, a liposome, a suppository, a plaster, a band-aid, a retard capsule, a powder, or a slow release formulation.

24. Pharmaceutical composition according to claim 22 or 23, wherein the diluent is water, a buffer, a buffered salt solution or a salt solution.

25. Pharmaceutical composition according to claims 22 to 24, wherein the carrier is selected from the group consisting of cocoa butter and vitebesole.

26. Use of a biological active fraction according to claim 21 or of a pharmaceutical composition according to claims any one of 22 to 25 for the production of a medicament for the improvement of cognitive and/or learning skills in particular improvement of the long term memory.

27. Use of a biological active fraction according to claim 21 or of a pharmaceutical composition according to any one of claims 22 to 25 for the production of a medicament for the treatment of seizures, in particular epileptic seizures, tremor, in particular associated with Parkinson's disease, proliferative diseases and cardiovascular disease, in particular myocardial in fraction and apoplexy.

28. Use according to claim 26, wherein the proliferative disease is selected from the group consisting of malignomas of the gastrointestinal or colorectal tract, the liver, the pancreas, the kidney, the bladder, the thyroid, the prostate, the endometrium, the cervix, the ovary, the uterus, the testes, the skin, the oral cavity; melanoma; dysplastic oral mucosa; invasive oral cancers; small cell and non-small cell lung carcinomas; mammary tumors, in particular hormone-dependent breast cancers and hormone independent breast cancers; transitional and squamous cell cancers; neurological malignancies including neuroblastomas, gliomas, astrocytomas, osteosarcomas, meningiomas; soft tissue sarcomas; hemangioamas and endocrinological tumors, in particular pituitary adenomas, pheochromocytomas, paragangliomas, haematological malignancies , in particular lymphomas and leukemia.

29. Use according to claims 26 to 28, wherein the medicament is administered to a subject in need of treatment in an amount ranging from 0.1 to 50 mg/kg body weight, preferably ranging from 1 to 10 mg/kg body weight.
